(19) Europäisches Patentamt
European Patent Office
Office européen des brevets

(11) **EP 2 491 898 B1**

(12) **EUROPEAN PATENT SPECIFICATION**

(45) Date of publication and mention
of the grant of the patent:
**11.02.2015 Bulletin 2015/07**

(51) Int Cl.:
***A61F 5/451*** *(2006.01)* *A61F 13/42* *(2006.01)*
***A61F 13/84*** *(2006.01)*

(21) Application number: **10824857.6**

(22) Date of filing: **15.10.2010**

(86) International application number:
**PCT/JP2010/068121**

(87) International publication number:
**WO 2011/049008 (28.04.2011 Gazette 2011/17)**

(54) **DEFECATION DETECTION DEVICE**

STUHLENTLEERUNGSDETEKTOR

DISPOSITIF DE DÉTECTION DE DÉFÉCATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.10.2009 JP 2009244822**

(43) Date of publication of application:
**29.08.2012 Bulletin 2012/35**

(73) Proprietor: **Uni-Charm Corporation
Ehime 799-0111 (JP)**

(72) Inventors:
• **SUZUKI, Miou
Kanonji-shi
Kagawa 769-1602 (JP)**
• **FUJIOKA, Yoshihisa
Kanonji-shi
Kagawa 769-1602 (JP)**

(74) Representative: **Eke, Philippa Dianne
Saunders & Dolleymore LLP
9 Rickmansworth Road
Watford Hertfordshire WD18 0JU (GB)**

(56) References cited:
WO-A1-2009/052496     WO-A2-2010/076679
JP-A- 2001 318 067     JP-A- 2008 043 501
JP-A- 2008 048 912     JP-A- 2009 056 285

**Description**

Technical Field

The present invention relates to a defecation detection apparatus.

Background Art

A device for detecting defecation based on a signal output from a temperature sensor placed in a diaper, for example, is already known (see Patent Document 1, for example). Such a device detects that feces have been discharged by detecting a temperature rise from a signal output from a single temperature sensor placed in a diaper.

Prior Art Documents

Patent Documents

[Patent Document 1] JP 2002-301098A JP 2008 048 912 is considered to represent the closest prior art.

Summary

Problems to be Solved by the Invention

The temperature in a diaper changes, not only with defecation and urination, but also with a movement of the body of the wearer of the diaper. Therefore, there is the possibility that discharge of feces may not be detected correctly.
In view of the above problem, it is an objective of the present invention to provide a defecation detection apparatus that can detect defecation more correctly.

Means for Solving the Problems

To attain the above objective, according to a main aspect of the present invention, a defecation detection apparatus is provided as defined in claim 1.
Other features of the present invention will become apparent from the following description taken in connection with the accompanying drawings.

Effects of Invention

According to the present invention, a defecation detection apparatus that can detect defecation more correctly is achieved.

Brief Description of Drawings

[FIG. 1] FIG. 1 is a view showing a configuration of an automatic urine disposal apparatus of this embodiment.
[FIG. 2] FIG. 2 is a plan view showing the inner side of a urine absorption member.
[FIG. 3] FIG. 3 is a view showing a section taken along line A-A in FIG. 2.
[FIG. 4] FIG. 4 is a view showing a section taken along line B-B in FIG. 2.
[FIG. 5] FIG. 5 is a plan view of an electrode section.
[FIG. 6] FIG. 6 is a cross-sectional view taken along line C-C in FIG. 5.
[FIG. 7] FIG. 7 is a cross-sectional view taken along line D-D in FIG. 5.
[FIG. 8] FIG. 8 is a cross-sectional view taken along line E-E in FIG. 5.
[FIG. 9] FIG. 9 is a plan view of the electrode section in a state where an insulating sheath is partly removed to expose power supply electrodes.
[FIG. 10] FIG. 10 is a view for explaining temperature changes at the time of urination and at the time of defecation.
[FIG. 11] FIG. 11 is a diagrammatic view for explaining noise removal processing.
[FIG. 12] FIG. 12 is a view showing a detection method of urination and defecation in the automatic urine disposal apparatus.

Description of Embodiments

At least the following matters will become apparent with the following description taken in connection with the accompanying drawings.

The defecation detection apparatus includes:

a feces receiving member placed to face the body of a wearer to receive discharged feces;
a first temperature sensor placed at a defecation position of the feces receiving member where discharged feces are received;
a second temperature sensor placed at a non-defecation position of the feces receiving member where discharged feces are not received; and
a control section that detects whether the discharged feces is present or not based on a signal output from the first temperature sensor and a signal output from the second temperature sensor.

In the above defecation detection apparatus, when feces are discharged, the first temperature sensor placed at the defecation position of the feces receiving member that receives discharged feces comes into contact with the feces, and senses a sharp rise in temperature. In the meantime, the second temperature sensor placed at a non-defecation position of the feces receiving member does not come into contact with feces when feces are discharged, and therefore the second temperature sensor senses no sharp rise in temperature due to the discharged feces. Moreover, the first and second temperature sensors, placed on the single feces receiving member, undergo roughly the same influence of a temperature change due to a cause other than defecation in the space between the feces receiving member and the body. Thus, the control section can detect whether defecation is present or not based on the signal output from the first temperature sensor that includes a temperature change due to defecation and a temperature change due to a cause other than defecation and the signal output from the second temperature sensor that includes a temperature change due to a cause other than defecation, permitting more correct detection of defecation. The defecation position as used herein refers to a position of the feces receiving member at which feces accumulate when a bedridden person who requires care and wears the feces receiving member discharges feces, for example, which specifically corresponds to a position facing the anus of the bedridden person and a position on the back side of the anus. The non-defecation position as used herein refers to a position of the feces receiving member other than the defecation position, for example.

In the defecation detection apparatus described above, desirably, the control section detects whether the discharged feces is present or not based on a signal obtained by subtracting the signal output from the second temperature sensor from the signal output from the first temperature sensor.

In the above defecation detection apparatus, the signal used by the control section to detect defecation is obtained by subtracting the signal output from the second temperature sensor from the signal output from the first temperature sensor. This makes it possible to detect defecation more correctly from a more correct signal that does not include any temperature change due to a cause other than defecation.

In the defecation detection apparatus described above, desirably, the second temperature sensor is placed at a position facing the groin or a position between the position facing the groin and the defecation position when the feces receiving member faces the body.

Since persons who need defecation detection are those who require nursing care such as bedridden elderly persons, for example, the defecation detection apparatus is used for such persons requiring care when lying on the bed. When a person requiring care discharges feces when lying on the bed, the feces will accumulate at a position lower than his or her body, i.e., at a position on the back side of the body. Also, it is desirable that the second temperature sensor capable of detecting a temperature change due to any cause other than defecation be placed at a non-defecation position that is as close to the first temperature sensor as possible and yet kept from contact with feces. Therefore, by placing the second temperature sensor at a position facing the groin or a position somewhere between the position facing the groin and the defecation position, it is possible to detect a temperature change due to any cause other than defecation in the first temperature sensor more reliably without subjecting the second temperature sensor to feces. Thus, defecation can be detected more correctly.

In the defecation detection apparatus described above, desirably, the first temperature sensor and the second temperature sensor are formed on a single insulating synthetic resin film.

In the above defecation detection apparatus, since the first and second temperature sensors are formed on the single insulating synthetic resin film, they can be easily attached to the film without the necessity of attaching the first temperature sensor and the second temperature sensor separately. Also, since it is on the insulating synthetic resin film, which is thin and flexible, that the first and second temperature sensors are formed, the user can use the resultant member without any discomfort.

The defecation detection apparatus described above desirably further includes a notification section that makes a notification that feces have been discharged, wherein the control section actuates the notification section when detecting that feces have been discharged.

In the above defecation detection apparatus, when defecation has occurred, it is possible to notify the caregiver, for example, of the defecation.

The defecation detection apparatus described above desirably further includes a urine detection section that detects urine discharged into the feces receiving member.

In the above defecation detection apparatus, not only defecation but also urination can be detected.

In the defecation detection apparatus described above, desirably, the urine detection section includes paired electrodes formed on the insulating synthetic resin film with spacing therebetween, and urine is detected based on a change in voltage between the paired electrodes caused by the discharged urine.

In the above defecation detection apparatus, since the urine detection section includes the paired electrodes formed on the insulating synthetic resin film with spacing therebetween, the function of detecting urine can be achieved at low cost. Also, since the paired electrodes are formed on the thin, flexible insulating synthetic resin film, the user can use the resultant member without any discomfort. Moreover, since the conductivity of the paired electrodes with spacing therebetween increases with the presence of urine, detection of urine can be ensured by detecting urine based on a change in the voltage between the electrodes.

The defecation detection apparatus described above desirably further includes a urine suction device detachably attached to the feces receiving member for sucking urine discharged in the feces receiving member, wherein the control section actuates the urine suction device to suck urine in the feces receiving member when the urine detection section has detected urine.

In the above defecation detection apparatus, since the urine suction device capable of sucking urine is provided, urine is sucked when urination is detected, permitting continuous use of the apparatus.

===Configuration of Automatic Urine disposal apparatus===

An automatic urine disposal apparatus as an example of the defecation detection apparatus will be described with reference to the accompanying drawings.

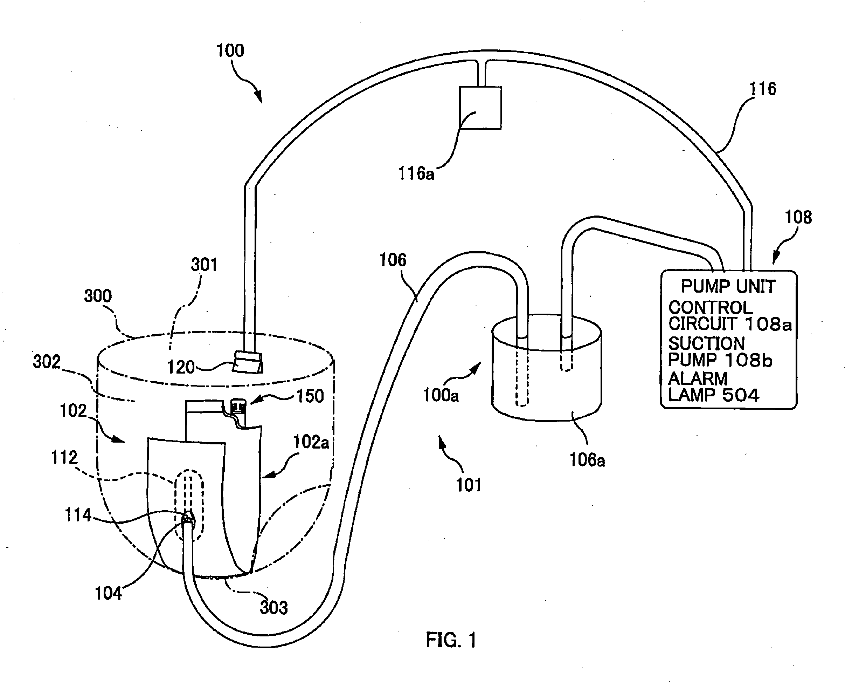

FIG. 1 is a view showing a configuration of an automatic urine disposal apparatus 100 of this embodiment. The automatic urine disposal apparatus 100 includes: a urine absorption member 102 shown as a partly cutaway figure; and a controller 101 provided with a vacuum suction device 100a as a urine suction device, to which the urine absorption member 102 is attached detachably. The urine absorption member 102 has an inner-surface side facing the skin of the wearer (not shown) and an outer-surface side opposite to the inner-surface side facing the clothing of the wearer, and is worn together with a pair of underpants 300 shown by phantom lines in FIG. 1 as the clothing, to allow the inner surface to be in close contact with the skin. The underpants 300 have a front waist region 301, a back waist region 302, and a crotch region 303, and are preferably made of, for example, a meshed cloth so that the outer-surface side can be easily seen through the underpants. Note that the urine absorption member 102 can be worn with, not only the underpants 300 as illustrated, but also other appropriate members such as an open diaper secured with tapes, a pants-type diaper, a diaper cover, underpants for incontinence patients, etc.

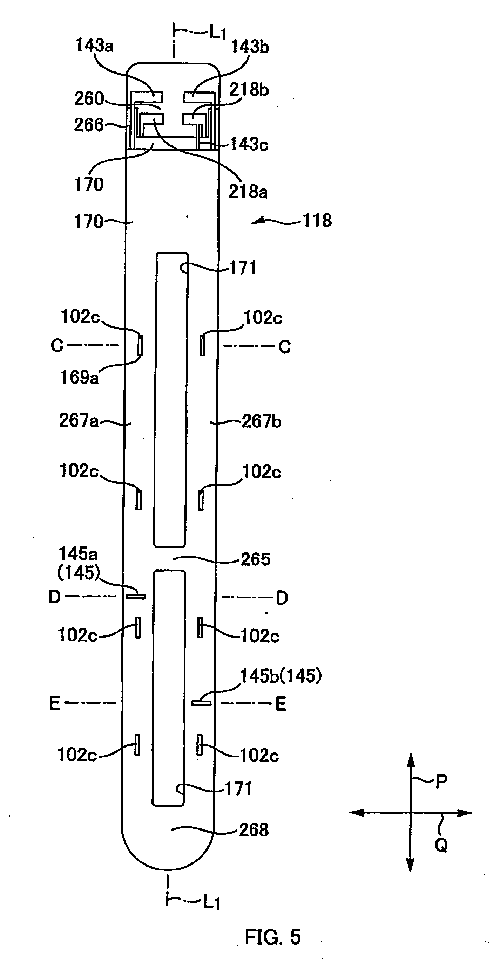

The automatic urine disposal apparatus 100 is an apparatus that can collect urine discharged by the wearer in the urine absorption member 102 and dispose of the collected urine. The urine absorption member 102 has a container section 102a and a detection section 150. The container section 102a faces the skin of the wearer near the urethral opening and can receive discharged urine. The detection section 150 includes a urine detection section 102b that detects discharge of urine and thermistors 145 as a feces detection section that detects feces (see FIG. 5). The vacuum suction device 100a includes a joint member 104 for connection to the container section 102a, a urine guide tube 106, a urine tank 106a, a pump unit 108, electrical wiring 116, etc.

The pump unit 108 includes a control circuit 108a as a control section that processes electric signals sent from the detection section 150 via the electrical wiring 116, a suction pump 108b, the drive of which is controlled by the control circuit 108a, etc. The urine guide tube 106 is connected via the joint member 104 to a urine drainage port 114 formed on the peripheral wall of a container 112 of the container section 102a of the urine absorption member 102. A clip 120 is attached to the end of the electrical wiring 116 extending from the pump unit 108, to electrically connect the electrical wiring 116 to urine detection electrodes 218a and 218b (see FIG. 5) as a pair of electrodes constituting the urine detection section 102b of the detection section 150 and power supply electrodes 143a, 143b, and 143c for supplying power to the thermistors 145. In such an automatic urine disposal apparatus 100, when urine is discharged, a detection signal is sent from the urine detection section 102b to the pump unit 108, which then actuates the suction pump 108b to suck the air in the urine tank 106a, thereby sucking the urine into the container 112 and further sucking the urine in the container 112 via the joint member 104 and the urine guide tube 106, to be collected in the urine tank 106a. In addition, signals output from the thermistors 145 placed in the urine absorption member 102 are sent to the pump unit 108. The control circuit 108a of the pump unit 108 allows an alarm lamp 504 as a notification section to blink based on the received signals, thereby notifying a caregiver of the presence of feces.

As shown in FIG. 1, when the urine absorption member 102 is worn, the clip 120 is on the belly side. The urine absorption member 102 is worn in the following manner: most of the container 112 of the urine absorption member 102 extends in the vertical direction on the front side of the wearer's body with the inside thereof facing the urethral opening and its surrounding skin of the wearer, and the lower end portion extends while curving gradually along the inner surface of the crotch region 303 toward the anus to reach the back of the body. In particular, since the urine absorption member 102 is preferably worn by a bedridden person, the urine absorption member 102 is formed to rest on a portion of the crotch region 303 closer to the back waist region 302. Therefore, the urine absorption member 102 can receive not only urine but also discharged feces.

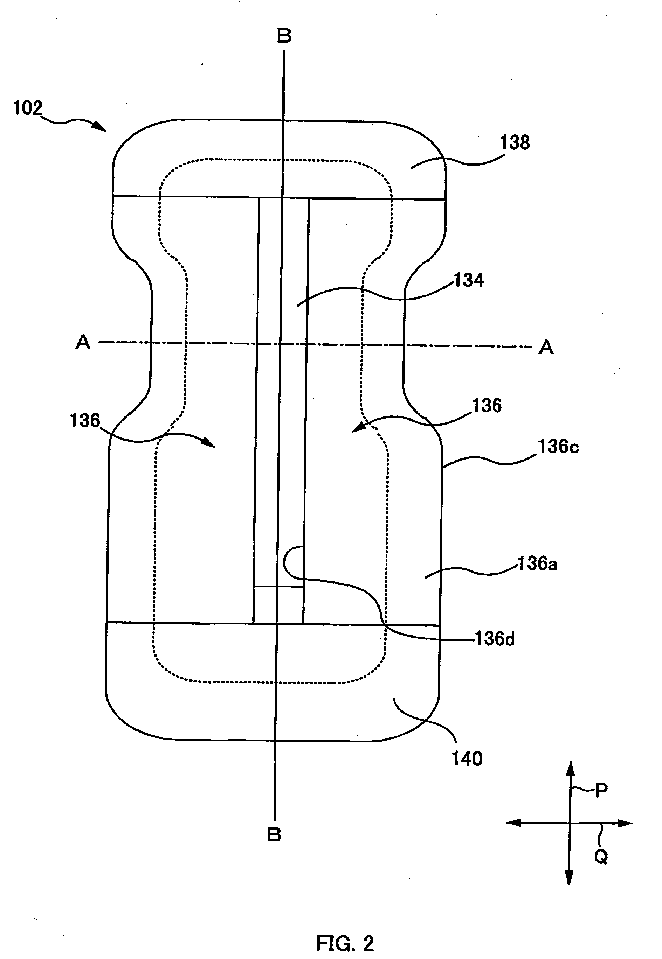

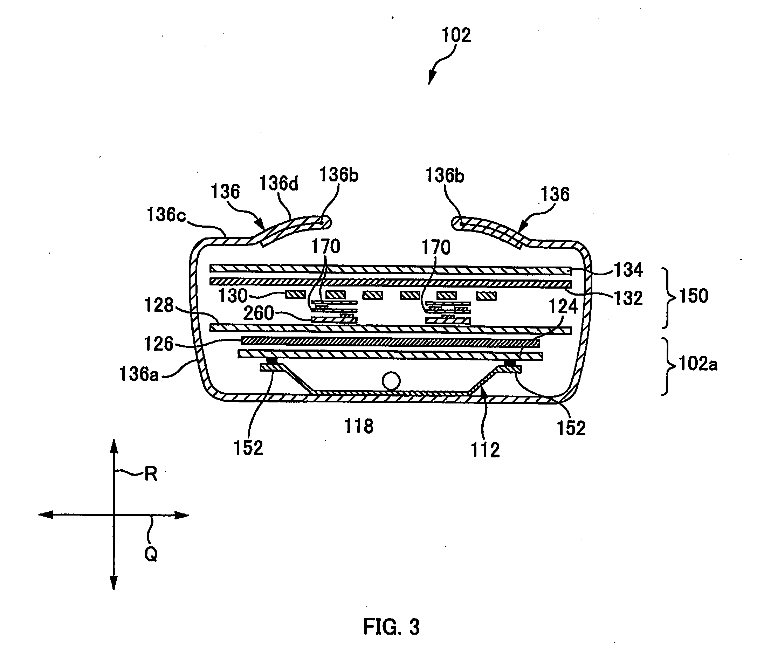

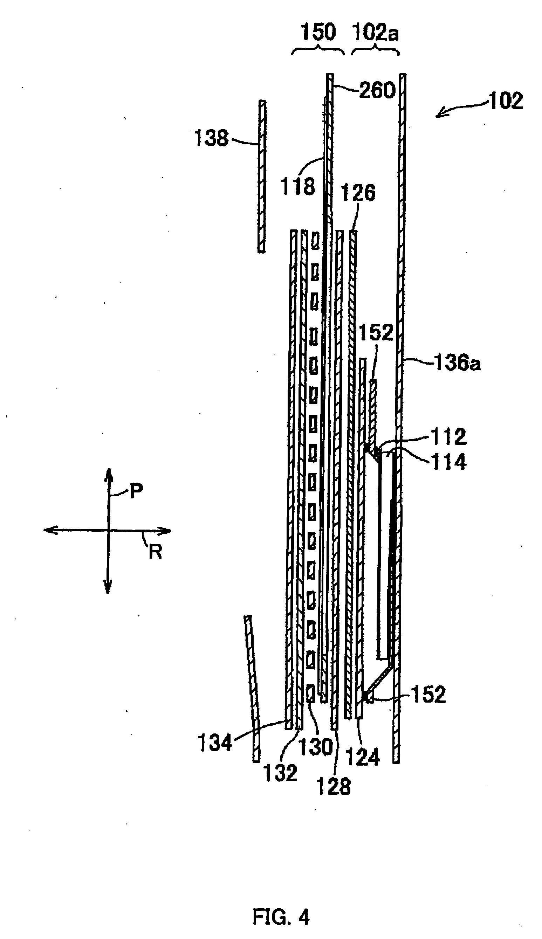

FIG. 2 is a plan view showing the inner-surface side of the urine absorption member 102, FIG. 3 is a view showing a section taken along line A-A in FIG. 2, and FIG. 4 is a view showing a section taken along line B-B in FIG. 2. Note that, in FIGS. 3 and 4, components that should be placed one upon another in a direction R of the thickness of the urine absorption member 102 are shown as if they are apart from one another with some exceptions. The thickness direction R is also the direction of the depth of the container section 102a.

The urine absorption member 102 has a length direction P to be aligned with the front-back direction of the wearer's body and a width direction Q orthogonal to the length direction P, where the width is large in portions at and near both ends in the length direction P and small in the center portion. The urine absorption member 102 also has the thickness direction R, and on the upper side of the container 112 as viewed from FIG. 3 (on the skin side when worn), placed one upon another are a plurality of sheet-like members including a liquid-permeable, substantially non-air-permeable sheet 124, a diffusion sheet 126, a cushion sheet 128, an electrode section 118, a spacer 130, a filter 132, and a liquid-permeable, skin-contact sheet 134 in this order from the bottom side (clothing side when worn) upward in the thickness direction R. A pair of leakage barriers 136 lie on the skin-contact sheet 134. The substantially non-air-permeable sheet 124 and the diffusion sheet 126 are integrated with the container 112, to form the container section 102a. The cushion sheet 128, the electrode section 118, the spacer 130, the filter 132, and the skin-contact sheet 134 lie one upon another, to form the detection section 150. In this embodiment, the portion of the urine absorption member 102 excluding the electrode section 118 corresponds to the feces receiving member for receiving feces.

The container 112, in the shape of a tray, is formed of a flexible, elastic member such as a flexible polyethylene and silicone rubber and has a flexibility permitting bending both in the length direction P and the width direction Q, but is built to resist deformation due to a negative pressure acting when the suction pump 108b sucks urine. The substantially non-air-permeable sheet 124 is bonded to a peripheral flange 152 of the container 112 at a position 112a by adhesion or welding. The depth direction of the container 112 is the same as the thickness direction R.

The substantially non-air-permeable sheet 124, which has high liquid permeability but hardly, or does not at all, permeate the air, covers the top opening of the container 112 as shown in FIG. 3. The container 112 having the substantially non-air-permeable sheet 124 easily goes negative in pressure when the suction pump 108b of the pump unit 108 is actuated, permitting prompt suction of urine. As the substantially non-air-permeable sheet 124, it is possible to use an SMS nonwoven fabric formed of a 22 g/m$^2$ spunbonded nonwoven fabric, a 10 g/m$^2$ melt-blown nonwoven fabric, and a 22 g/m$^2$ spunbonded nonwoven fabric, preferably subjected to hydrophilic treatment with a surfactant. The air permeability of the substantially non-air-permeable sheet 124 as measured according to method A of the air permeability measurement methods defined in JIS L 1096 6.27.1 is in the range of 0 to 100 cc/cm$^2$/sec., preferably in the range of 0 to 50 cc/cm$^2$/sec., in its wet state, and in the range of 20 to 200 cc/cm$^2$/sec., preferably in the range of 20 to 100 cc/cm$^2$/sec., more preferably in the range of 20 to 50 cc/cm$^2$/sec., in its dry state. The wet state at the measurement of the air permeability is defined as the state where the water content of the substantially non-air-permeable sheet 124 as calculated in equation (1) below is 100% or more, and the dry state is defined as the state of the substantially non-air-permeable sheet 124 observed after having been left to stand in a 20°C, 50%RH room for 24 hours or more.

```
    Water content = (wet-state sheet weight - dry-state sheet
weight) /       (dry-state sheet weight)      .   .   .
Equation (1)
```

The diffusion sheet 126, which is formed of a liquid-permeable sheet strip such as a nonwoven fabric including hydrophilic fibers such as rayon fibers, for example, is used to diffuse urine, when discharged, over the surface (skin side) of the substantially non-air-permeable sheet 124 promptly to put the substantially non-air-permeable sheet 124 into the wet state over a wide area. With the substantially non-air-permeable sheet 124 being in the wet state, it is easy to suck the urine into the container 112 by rendering the inside of the container 112 negative in pressure. It is preferable that the diffusion sheet 126 be bonded to the substantially non-air-permeable sheet 124 sporadically so as not to impair the liquid permeability of either.

The cushion sheet 128, which is formed of a liquid-permeable sheet strip such as a thermal bonded nonwoven fabric having a basis weight of 20 to 30 g/m$^2$, for example, allows urine to permeate therethrough promptly, and prevents backflow of urine present in the diffusion sheet 126 and the substantially non-air-permeable sheet 124 to the electrode section 118. Also, by previously placing the sheet-like members such as the electrode section 118, the spacer 130, the filter 132., etc. one upon another on the cushion sheet 128, the cushion sheet 128 serves as a carrier member for placing these sheet-like members in a predetermined portion of the urine absorption member 102 in the process of manufacturing the urine absorption member 102. It is preferable that the cushion sheet 128 be bonded to the diffusion sheet 126 sporadically so as not to impair the liquid permeability of either.

The electrode section 118 has mounted thereon thin-film thermistors (hereinafter simply referred to as thermistors) for detecting feces, and includes electrodes in a predetermined shape for detecting urine and electrodes for supplying electric power to the thermistors printed on a synthetic resin film with conductive ink. The details of the electrode section 118 will be described later. The electrode section 118 can be bonded to the cushion sheet 128. As the thermistors 145 suitable for the automatic urine disposal apparatus 100 of this embodiment, thermistors that are small in heat capacity and susceptible to the surrounding temperature are preferable. An example of such thermistors is thermistors ET-103 manufactured by SEMITEC Corp.

The spacer 130, thickest among the sheet-like members of the detection section 150, is formed of a net-shaped liquid-permeable sheet strip. In the urine absorption member 102, some urine may remain in the skin-contact sheet 134 after suction of urine, leaving the skin-contact sheet 134 in the wet state with the remaining urine. Such a skin-contact sheet 134 may come into contact with the electrode section 118 directly or indirectly under the action of the pressure from the body, etc., causing a malfunction of the automatic urine disposal apparatus 100. The spacer 130 is a member provided to secure spacing between the electrode section 118 and the filter 132 in the thickness direction R, thereby preventing such a malfunction, and has water repellency with no urine absorption capability, has air permeability and liquid permeability higher than the substantially non-air-permeable sheet 124, and does not change in thickness under the pressure from the body. Such a spacer 130 can be formed of a net having a thickness of 0.5 to 1 mm made of a flexible synthetic resin such as ethylenevinyl acetate, and is preferably bonded to the cushion sheet 128 so as not to impair the liquid permeability of either.

The filter 132 is provided to prevent occurrence of an event that a solid content included in urine may attach to the electrode section 118 causing permanent energization of the electrode section 118, and is formed of a sheet strip, more preferably a nonwoven fabric, having air permeability and liquid permeability higher than the substantially non-air-permeable sheet 124. The filter 132 can be bonded to the spacer 130 so as not to impair the liquid permeability of either.

The skin-contact sheet 134, placed on the surface (skin side) of the filter 132, comes into contact with the wearer's skin facing the urethral opening and its surrounding skin of the wearer when the urine absorption member 102 is worn. Such a skin-contact sheet 134 is formed of a sheet strip having flexibility and liquid permeability, such as a thermal bonded nonwoven fabric having a basis weight of 15 to 25 g/m$^2$, for example. Like the cushion sheet 128, the skin-contact sheet 134 allows urine to permeate therethrough instantaneously at the initial stage of urination, and is preferably bonded to the filter 132 sporadically so as not to impair the liquid permeability of either. The skin-contact sheet 134 may be hydrophilic in some cases and water-repellent in other cases.

The leakage barriers 136 on the right and left sides as a pair as shown in FIGS. 2 and 3 can prevent urine from flowing on the skin-contact sheet 134 in the width direction Q leaking sideways from the urine absorption member 102. In the leakage barriers 136 shown in FIG. 3, while outer edge portions 136c located in the outer part of the urine absorption member 102 are bonded to the skin-contact sheet 134, inner edge portions 136d located in the inner part thereof are not bonded to the skin-contact sheet 134, but have elastic members 136b such as rubber threads attached thereto in the stretched state in the length direction P. A sheet 136a constituting the pair of leakage barriers 136 covers the bottom of the container 112. When being worn, the urine absorption member 102 bends in the length direction P as shown in FIG. 1, causing the elastic members 136b to shrink and thus the inner edge portions 136d of the leakage barriers 136 to stand upward away from the skin-contact sheet 134. It is preferable that the sheet 136a forming the leakage barriers 136 be liquid impermeable, and for this, a flexible thermoplastic synthetic resin film, a composite sheet of this film and a nonwoven fabric, etc. can be used. When the urine absorption member 102 is viewed from the top (see FIG. 2), the top and bottom end portions of the leakage barriers 136 are covered with first and second end sheets 138 and 140, respectively.

FIG. 5 is a plan view of the electrode section 118 shown in FIGS. 2, 3, and 4. The electrode section 118 includes: an insulating film 260 formed of a synthetic resin film; the paired urine detection electrodes 218a and 218b formed on one surface of the film 260; two thermistors and the power supply electrodes 143a, 143b, and 143c for supplying power to the thermistors formed on the same surface of the film 260; and an insulating sheath 170 covering most of these electrodes 218a, 218b, 143a, 143b, and 143c.

The film 260, in the shape of a strip extending in the length direction P, has two rectangular openings 171 formed by cutting off portions that are elongated in the length direction P and are located in the center in the width direction Q. Such a film 260 has: a top end portion 266 in the upper part of FIG. 5 to be gripped with the clip 120; side portions 267a and 267b on both sides of the center line L1-L1 bisecting the width of the electrode section 118 extending below the top end portion 266; a bottom end portion 268 continuing from the side portions 267a and 267b in the lower part; and a connecting portion 265 for connecting the side portions 267a and 267b to each other at a position between the top end portion 266 and the bottom end portion 268. On the top end portion 266 of the film 260, the ends of the urine detection electrodes 218a and 218b and the ends of the power supply electrodes 143a, 143b, and 143c are exposed. Also, the insulating sheath 170 has eight uncoated portions 169a on the side portions 267a and 267b. Such uncoated portions 169a are arranged in two lines in the length direction P at appropriate intervals with two each aligned in the width direction Q. The urine detection electrodes 218a and 218b are partly exposed from the uncoated portions 169a to allow the electrodes 218a and 218b to get wet with urine.

More specifically, the urine detection electrodes 218a and 218b are formed on one surface of the film 260, extending from the top end portion 266 to the bottom end portion 268 through the side portions 267a and 267b. Such urine detection electrodes 218a and 218b then turn on the bottom end portion 268 inwardly in the width direction Q, extend upward along the opening 171 closer to the bottom end portion 268, and are connected to each other on the connecting portion 265. The portions of the urine detection electrodes 218a and 218b from the turn on the bottom end portion 268 to the connection on the connecting portion 265 serve as a break detection circuit 250 to be described later. The urine detection electrodes 218a and 218b are covered with the insulating sheath 170 except for the ends on the top end portion 266 that are to be connected to the pump unit 108 when gripped with the clip 120 and the uncoated portions 169a.

The power supply electrodes 143a, 143b, and 143c are formed in the surface portion of the insulating sheath 170, extending from the top end portion 266 through the side portions 267a and 267b. The power supply electrodes 143a and 143b have their bottom ends at positions closer to the top end portion than the urine detection electrodes 218a and 218b, and extend on the side portions 267a and 267b on the outer side of the urine detection electrodes 218a and 218b in the width direction of the electrode section 118. The power supply electrode 143c is formed to overlap one of the exposed urine detection electrodes 218a and 218b on the top end portion 266, extends on the inner side of the overlapped urine detection electrode 218a or 218b (218b in the illustrated example) on the side portion 267b, and is branched into two on the connecting portion 265. One of the portions branched at the connecting portion 265 extends straight in the length direction P of the electrode section 118, and the other extends on the connecting portion 265 and then extends between the urine detection electrode 218a and the opening 171 closer to the bottom end portion 268.

The power supply electrode 143a and the branched portion of the power supply electrode 143c are formed so that the ends thereof closer to the bottom end portion 268 in the side portion 267a reach a position slightly backward from the groin that is the center of the urine absorption member 102 in the body front-back direction when the urine absorption member 102 is worn. The power supply electrode 143b and the straight portion of the power supply electrode 143c are formed so that the ends thereof closer to the bottom end portion 268 reach a position backward from the position facing the anus of the wearer of the urine absorption member 102. The power supply electrodes 143a, 143b, and 143c are covered with the insulating sheath 170 excluding the ends on the top end portion 266 and the ends closer to the bottom end portion 268. Note that the portions of the urine detection electrodes 218a and 218b corresponding to the uncoated portions 169a are exposed without being covered with the two-layer insulating sheath 170.

The thermistors 145 are placed to extend between the end of the power supply electrode 143a closer to the bottom end portion 268 and the end of the branched portion of the power supply electrode 143c closer to the bottom end portion 268 and between the end of the power supply electrode 143b closer to the bottom end portion 268 and the end of the straight portion of the power supply electrode 143c closer to the bottom end portion 268. The surfaces of the thermistors 145 are covered with a protection sheet not shown.

Note that hereinafter the thermistor 145 at the position closer to the groin is referred to as a front-side thermistor 145a and the thermistor 145 at the position closer to the anus is referred to as a back-side thermistor 145b. Note also that the position of the front-side thermistor 145a corresponds to a non-defecation position free from receiving discharged feces because it is at a level above the anus when a person requiring nursing care as the wearer lies on his or her back, and the position of the back-side thermistor 145b corresponds to a defecation position receiving discharged feces because it is at a level below the anus. Therefore, the front-side thermistor 145a corresponds to the second temperature sensor and the back-side thermistor 145b corresponds to the first temperature sensor.

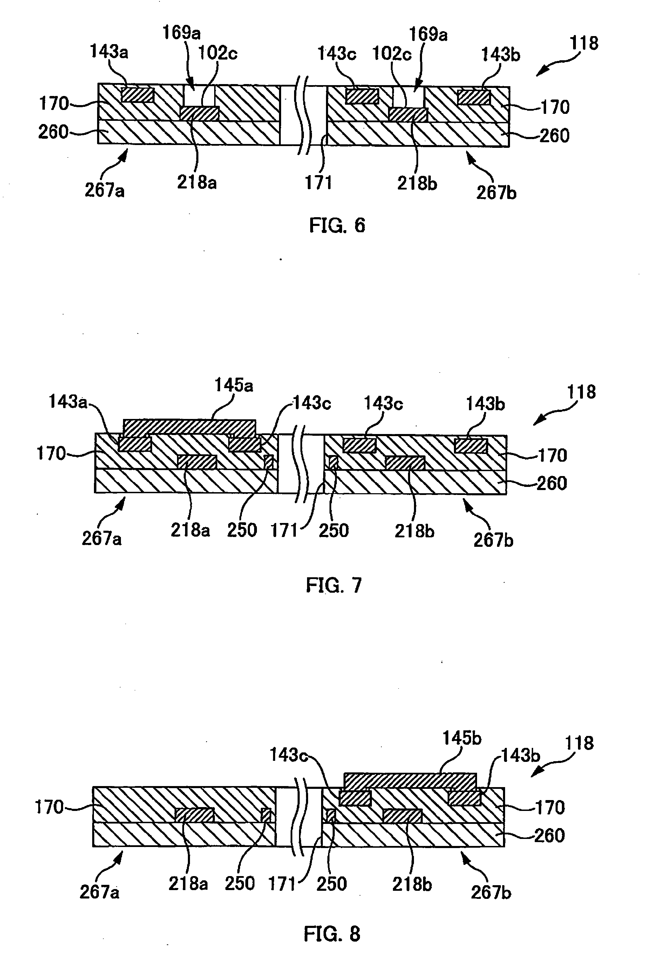

FIG. 6 is a cross-sectional view taken along line C-C in FIG. 5, showing exposure portions 102c of the urine detection electrodes 218a and 218b. In FIG. 6, the power supply electrodes 143a, 143b, and 143c are covered with the insulating sheath 170.

FIG. 7 is a cross-sectional view taken along line D-D in FIG. 5, showing the state where the front-side thermistor 145a is placed. In FIG. 7, the break detection circuit 250, the urine detection electrodes 218a and 218b, the power supply electrode 143b, and the power supply electrode 143c on the side portion 267b are covered with the insulating sheath 170, and the front-side thermistor 145a is connected to the power supply electrode 143a and the power supply electrode 143c on the side portion 267a.

FIG. 8 is a cross-sectional view taken along line E-E in FIG. 5, showing the state where the back-side thermistor 145b is placed. In FIG. 8, the break detection circuit 250 and the urine detection electrodes 218a and 218b are covered with the insulating sheath 170, and the back-side thermistor 145b is connected to the power supply electrode 143b and the power supply electrode 143c on the side portion 267b.

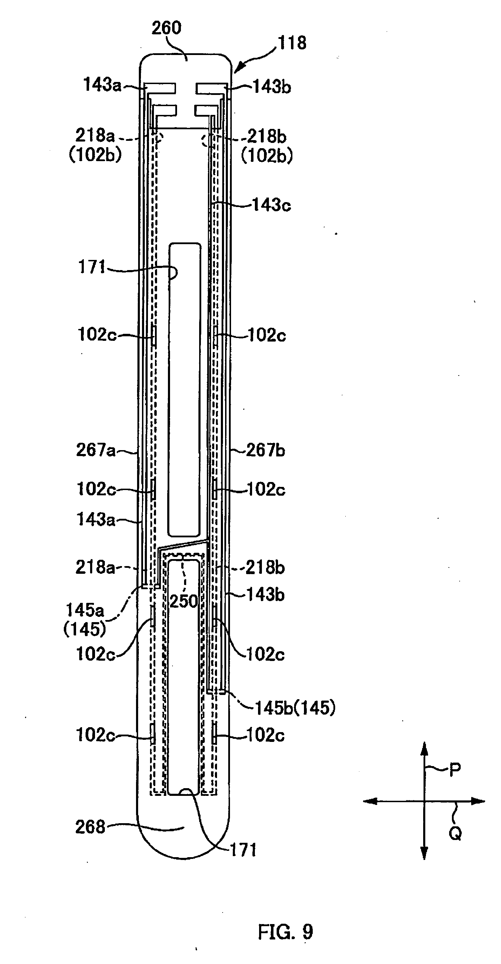

FIG. 9 is a plan view of the electrode section 118 in the state where part of the insulating sheath 170 is removed to expose the power supply electrodes 143a, 143b, and 143c. Formed on the side portions 267a and 267b of the film 260 are the paired urine detection electrodes 218a and 218b extending in parallel with spacing therebetween in the length direction P. These urine detection electrodes 218a and 218b are exposed in the uncoated portions 169a in FIG. 5. The break detection circuit 250 is formed between the urine detection electrodes 218a and 218b. The break detection circuit 250 is electrically connected to the bottom end portions of the urine detection electrodes 218a and 218b, and extends along the edge of the opening 171 as illustrated. Also formed on the side portions 267a and 267b of the film 260 are the power supply electrodes 143a and 143b for supplying power to the front-side thermistor 145a and the back-side thermistor 145b, extending in the length direction P on the outer side of the urine detection electrodes 218a and 218b, respectively, with spacing therebetween in the width direction Q. The thermistors 145 are provided at the ends of the power supply electrodes 143a and 143b.

In the electrode section 118, a polyester film having a thickness of 50 to 100 $\mu$m is preferably used as the film 260. The urine detection electrodes 218a and 218b can be formed by printing lines of a required shape on the film 260 with conductive ink and conductive paint. The conductive ink and the conductive paint include, as conductive materials, 3 to 7 wt% of carbon black, 10 to 30 wt% of artificial graphite such as carbon graphite, an appropriate amount of silver powder, etc., for example. The urine detection electrodes 218a and 218b are formed to have a width of 0.5 to 2 mm and a resistance of 150 k$\Omega$ or less, for example. The break detection circuit 250 can be formed by printing lines of a required shape on the film 260 with ink including 3 to 7 wt% of carbon black and 5 to 10 wt% of artificial graphite, for example. The break detection circuit 250, having a resistance value much higher than that of the urine detection electrodes 218a and 218b, is preferably formed to have a width of 0.3 to 1 mm and a resistance value of about 2 to 10 M$\Omega$. The power supply electrodes 143a, 143b, and 143c may be formed with ink and paint similar to those used for the urine detection electrodes 218a and 218b, or may be formed by vacuum evaporation of aluminum. The power supply electrodes 143a, 143b, and 143c are also formed to have a width of 0.5 to 2 m, and uncoated portions having an appropriate width are formed at the ends of the power supply electrodes 143a, 143b, and 143c to allow placement of the thermistors.

When the electrode section 118 and the controller 101 are electrically connected via the clip 120, a faint current is supplied from a power supply 116a (see FIG. 1) of the controller 101 to the urine detection electrodes 218a and 218b, and a level of electric power with which the thermistors are operable is supplied to the thermistors via the power supply electrodes 143a, 143b, and 143c.

The control circuit 108a of the pump unit 108 continuously or intermittently measures the electric resistance between the urine detection electrodes 218a and 218b or another physical amount equivalent to this electric resistance, as well as changes in the electric resistances output from the thermistors 145. Note that the urine detection electrodes 218a and 218b are connected to each other via the break detection circuit 250, and the control circuit 108a detects a faint current flowing through these electrodes and the circuit. If this current has not been detected for a predetermined time period or longer, it is determined that something unusual has occurred with the urine detection electrodes 218a and 218b, and an alarm is issued to the user of the automatic urine disposal apparatus 100.

When urine is discharged into the urine absorption member 102, the exposure portions 102c of the urine detection electrodes 218a and 218b are electrically connected to each other, changing the electric resistance between the urine detection electrodes 218a and 218b to a lower value. Interpreting this change as the presence of urine in the urine detection section 102b, i.e., as a signal indicating that urination has been detected, the control circuit 108a actuates the suction pump 108b. The degree of decrease of the electric resistance depends on various conditions of the urine absorption member 102, such as the exposed areas of the urine detection electrodes 218a and 218b in the uncoated portions 169a, for example. Therefore, in the illustrated urine absorption member 102, measures may be taken prior to use so that the electric resistance between the urine detection electrodes 218a and 218b easily decrease to 0.4 k$\Omega$ or less when urine is discharged, and continuation of the electric resistance of 0.4 k$\Omega$ or less for a predetermined time, e.g., 0.2 seconds, can be used as the specified resistance value, i.e., the threshold, for actuation of the suction pump 108b. The suction pump 108b preferably has the capability of completing the suction of urine with the urine absorption member 102 within one to two minutes. Using such a suction pump 108b, it is possible to determine that something unusual has occurred with the automatic urination apparatus 100 when the operation of the suction pump 108b has continued for three minutes or more, for example.

The thermistors 145a and 145b connected to the control circuit 108a of the pump unit 108 change their electric resistances with the temperature of the space between the urine absorption member 102 and the wearer's body. The control circuit 108a detects the electric resistances of the thermistors 145a and 145b at predetermined time intervals (e.g., intervals of one second). Moreover, the control circuit 108a detects temperature changes in the space between the urine absorption member 102 and the body from the change per second of the electric resistances based on the detected electric resistances. The front-side thermistor 145a placed at the non-defecation position detects a temperature change at the non-defecation position, and the back-side thermistor 145b placed at the defecation position detects a temperature change at the defecation position.

When the wearer discharges feces and the feces reach the defecation position to be close to or in contact with the back-side thermistor 145b, the electric resistance of the back-side thermistor 145b sharply increases because the temperature of the feces discharged from the body is higher than the body temperature.

The temperatures detected by the front-side thermistor 145a and the back-side thermistor 145b also change at occasions such as when the wearer discharges urine and when the wearer moves his or her body. When the wearer discharges urine, in particular, the electric resistance of the back-side thermistor 145b sharply increases, as it does when feces are discharged. It is therefore difficult to detect defecation with only the temperature change detected by the back-side thermistor 145b. This being the case, the present inventors have compared the temperature change detected by the back-side thermistor 145b at the time of urination to the temperature change detected by the back-side thermistor 145b at the time of defecation.

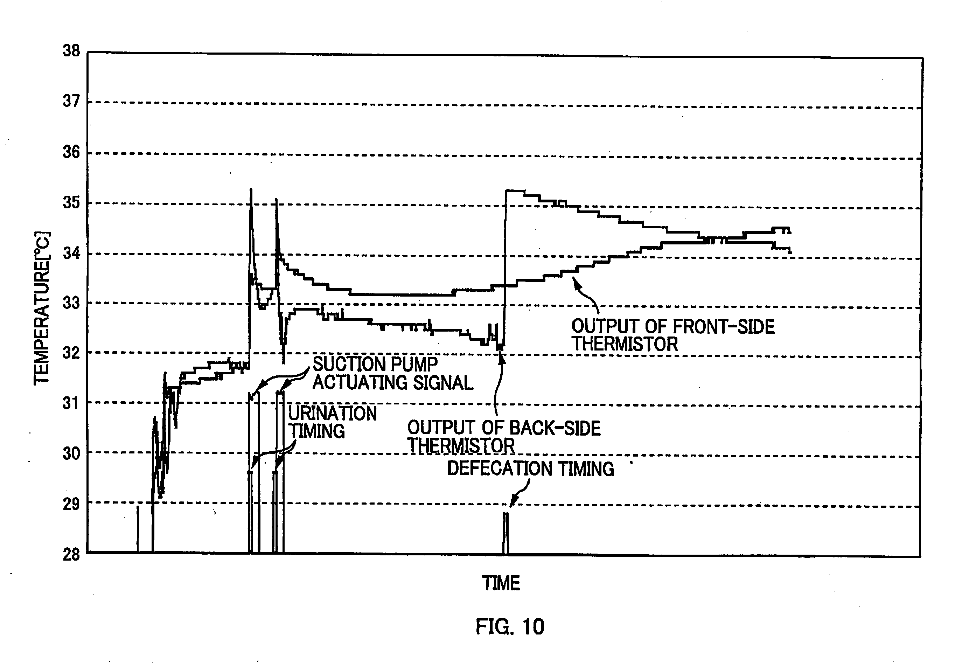

FIG. 10 is a view for explaining the temperature changes at the time of urination and at the time of defecation.

FIG. 10 shows the results of about one-hour continuous detection of the temperature changes of the front-side thermistor 145a and the back-side thermistor 145b of the urine absorption member 102 worn by a person who can recognize his or her defecation and urination: when discharging urine or feces, the wearer issues a signal S to the control circuit 108a to indicate the discharge. The suction pump 108b is actuated when urination is detected.

As illustrated, the temperature at the time of urination rises by 2 to 3°C in a few seconds and then falls by about 2°C in one to two minutes partly because the suction pump 108b is operating. The temperature at the time of defecation rises by 2 to 3°C in a few seconds as at the time of urination, but then falls only by about 0.3°C even after the lapse of about two minutes. In this way, while the temperature sharply rises both at the urination and at the defecation, the subsequent temperature change is different at the urination and at the defecation: the temperature falls sharply at the urination, but it falls gradually over time at the defecation. This is probably influenced by the suction of urine by the suction pump 108b, but also seems to be caused by the difference in heat capacity between urine and feces, and thus this difference is highly reliable. Thus, the present inventors have paid attention to the difference in the temperature fall at the urination and at the defecation. In other words, based on the change in the temperature after the rise of the temperature, when the fall of the temperature is continuing gradually within the range of a predetermined value (e.g., 1°C) after a predetermined time (e.g., 2 minutes), it is determined that defecation, not urination, has been detected.

In addition, as described above, the temperatures detected by the front-side thermistor 145a and the back-side thermistor 145b also change, for example, when the wearer moves his or her body. Therefore, in order to prevent erroneous detection due to a temperature change occurring when the body is moved, the automatic urine disposal apparatus 100 is provided with the front-side thermistor 145a. The front-side thermistor 145a, placed at a non-defecation position, is not likely to change its electric resistance due to defecation. On the other hand, the front-side thermistor 145a is placed to face the groin of the wearer at a non-defecation position and also at a position relatively near the defecation position. Therefore, when the wearer moves his or her body for example, causing a change in the temperature at a position between the urine absorption member 102 and the body, the output of the front-side thermistor 145a changes roughly similarly to that of the back-side thermistor 145b.

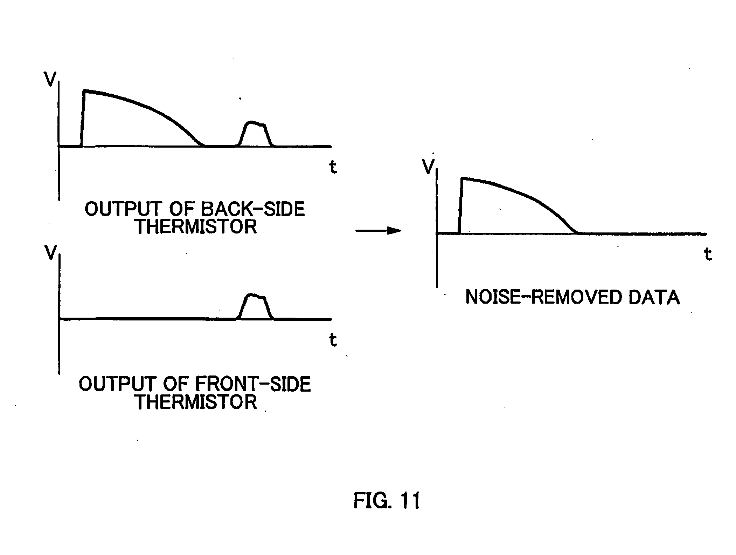

FIG. 11 is a diagrammatic view showing temperature changes detected by the front-side thermistor 145a and the back-side thermistor 145b. The upper-left view of FIG. 11 represents the temperature change detected by the back-side thermistor 145b, and the lower-left view of FIG. 11 represents the temperature change detected by the front-side thermistor 145a. While the signal output from the back-side thermistor 145b in the upper-left view indicates that a large temperature rise and fall have occurred in both the first part and the latter part, the signal output from the front-side thermistor 145a in the lower-left view indicates that a temperature rise and fall have occurred in synchronization with the latter temperature change in the signal output from the back-side thermistor 145b. In other words, since similar temperature changes have occurred at the defecation position and the non-defecation position in the latter part, it is presumed that the temperature change in the latter part must have occurred, not due to defecation, but due to a change in the temperature of the space between the urine absorption member 102 and the body caused by a movement of the wearer's body, etc. Therefore, in detection of defecation using the temperature of the back-side thermistor 145b, the control circuit 108a first executes noise removal processing by removing the temperature change detected by the front-side thermistor 145a.

===Feces Detection Method===

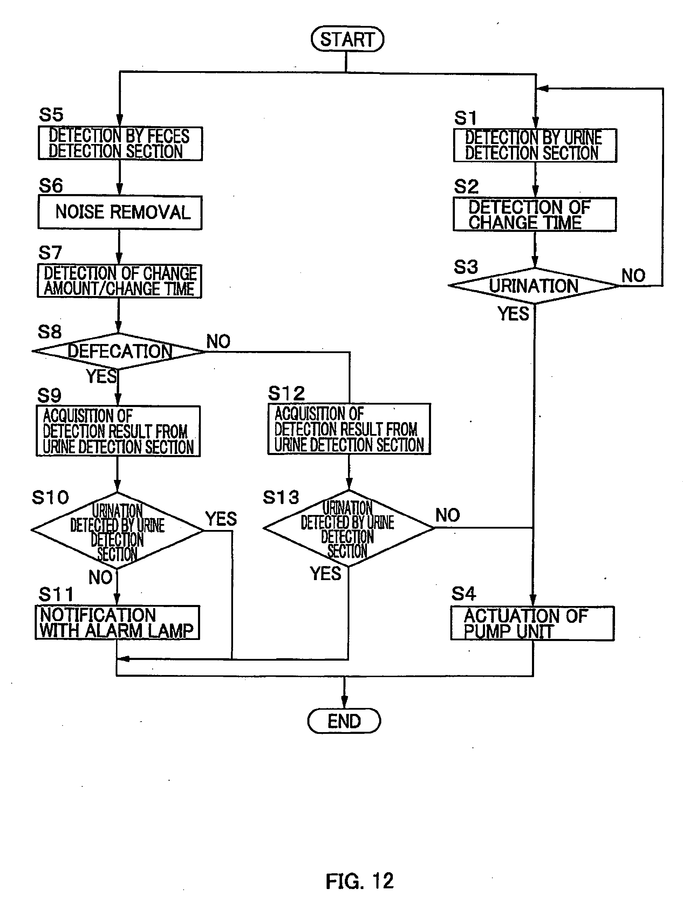

FIG. 12 is a view showing a detection method of urination and defecation by the automatic urine disposal apparatus 100.

As shown in FIG. 12, in the defecation detection method in the automatic urine disposal apparatus 100 of this embodiment, the control circuit 108a detects concurrently signals output from the urine detection electrodes 218a and 218b, the front-side thermistor 145a, and the back-side thermistor 145b. More specifically, the control circuit 108a of the pump unit 108 continuously or intermittently measures the electric resistance (impedance) between the urine detection electrodes 218a and 218b and the changes in the electric resistances output from the thermistors 145a and 145b.

When urine is discharged into the urine absorption member 102, the control circuit 108a detects that the electric resistance between the urine detection electrodes 218a and 218b has decreased (S1). With this detection of the decrease in the electric resistance between the urine detection electrodes 218a and 218b, the control circuit 108a detects the change in the electric resistance in a predetermined time, and, once stable output is resumed (S2), determines that this is the signal indicating detection of discharge of urine (S3), and actuates the suction pump 108b (S4).

Concurrently with the detection of urination, the control circuit 108a measures the changes in the electric resistances output from the front-side thermistor 145a and the back-side thermistor 145b synchronously at given time intervals. For example, assuming a signal obtained by measuring the change per second of the electric resistance for five minutes as one unit, the measurement is made by shifting the measurement start time by one second each.

When detecting a decrease in the electric resistance of the back-side thermistor 145b (S5), the control circuit 108a subtracts the signal indicating the change in electric resistance measured by the front-end thermistor 145a from the signal indicating the change in electric resistance measured by the back-side thermistor 145b, for the signal at and after the detection of the decrease in electric resistance, to remove a temperature change due to any cause other than defecation, i.e., noise (S6).

Then, the control circuit 108a detects the course of the change in electric resistance in the noise-removed signal, to calculate the rate of the temperature fall from the course of the change in electric resistance (S7). For example, for the noise-removed 5-minute signal, the control circuit 108a detects the temperature that has fallen in two minutes after the temperature rise has been detected based on the change in the electric resistance of the back-side thermistor 145b, to determine defecation or urination (S8). If the rate of rise of the electric resistance in the noise-removed signal, i.e., the rate of fall of the temperature, is slow, it is temporarily assumed that the signal is a signal indicating detection of discharge of feces. At this time, the detection result on whether urination is present or not based on the electric resistance value between the urine detection electrodes 218a and 218b is acquired (S9), and if discharge of urine has already been detected (S10), it is determined that the excrement is urine, not feces, giving high priority to the detection result by the urine detection electrodes 218a and 218b. In this case, since the suction pump 108b has already been actuated, there is no need to actuate the suction pump 108b at this stage.

If, after it is temporarily assumed that the signal is a signal indicating detection of discharge of feces based on the noise-removed signal (S8), the detection result on whether urination is present or not based on the electric resistance value between the urine detection electrodes 218a and 218b is acquired (S9), and in the case that no discharge of urine has been detected (S10), the control circuit 108a determines that the excrement is feces, and issues an alarm, etc. for urging for exchange of the urine absorption member 102 (S11).

If the rate of rise of the electric resistance in the noise-removed signal, i.e., the rate of fall of the temperature, is sharp, it is assumed that the signal is a signal indicating detection of discharge of urine (S8). At this time, the detection result on whether urination is present or not based on the electric resistance value between the urine detection electrodes 218a and 218b is acquired (S12), and in the case that discharge of urine has already been detected (S13), it is determined that the excrement is urine. In this case, since the suction pump 108b has already been actuated, it is unnecessary for the control circuit 108b to actuate the suction pump 108b at this stage. In the case that it is found that discharge of urine has not been detected from the detection result on whether urination is present or not based on the electric resistance value between the urine detection electrodes 218a and 218b (S13), the control circuit 108a actuates the suction pump 108b (S4).

In the automatic urine disposal apparatus 100 of this embodiment, when feces are discharged, the back-side thermistor 145b placed at the defecation position of the urine absorption member 102 receiving discharged feces comes into contact with the feces, and senses a sharp rise in temperature. On the other hand, the front-side thermistor 145a placed at a non-defecation position of the urine absorption member 102 does not come into contact with feces when feces are discharged, and therefore it senses no sharp rise in temperature due to discharged feces. In addition, the front-end thermistor 145a and the back-side thermistor 145b, which are placed on the single urine absorption member 102, undergo roughly the same influence of a temperature change due to a cause other than defecation in the space between the urine absorption member 102 and the wearer's body. Thus, the control circuit 108a can detect whether defecation is present or not based on the signal output from the back-side thermistor 145b that includes a temperature change due to defecation and a temperature change due to a cause other than defecation and a signal output from the front-side thermistor 145a that includes a temperature change due to a cause other than defecation and does not include a temperature change due to defecation, permitting more correct detection of defecation.

More specifically, since the control circuit 108a detects defecation based on the signal obtained by subtracting the signal output from the front-side thermistor 145a from the signal output from the back-side thermistor 145b, defecation can be detected more correctly from a more correct signal that does not include a temperature change due to any cause other than defecation.

In the defecation detection apparatus, it is desirable that the second temperature sensor be placed at a position facing the groin or a position between the position facing the groin and the defecation position when the feces receiving member faces the body.

Since persons who need defecation detection are those who require nursing care such as bedridden elderly persons, for example, the automatic urine disposal apparatus 100 is used for such persons requiring care while lying on the bed. When a person requiring care discharges feces when lying on the bed, the feces will accumulate at a position lower than his or her body, i.e., at the defecation position on the back side of the body. Also, it is desirable that the front-side thermistor 145a that can detect a temperature change due to any cause other than defecation be placed at a non-defecation position that is as close to the back-side thermistor 145b at the defecation position as possible and yet kept from contact with feces. Therefore, by placing the front-side thermistor 145a at a position facing the groin or a position somewhere between the position facing the groin and the defecation position, it is possible to detect a temperature change due to any cause other than defecation in the front-side thermistor 145a more reliably without subjecting the front-side thermistor 145a to feces. Thus, defecation can be detected more correctly.

In addition, since the front-side thermistor 145a and the back-side thermistor 145b are formed on the single insulating synthetic resin film 260, they can be easily attached to the film without the necessity of attaching the front-side thermistor 145a and the back-side thermistor 145b separately. Also, since it is on the thin, flexible, insulating synthetic resin film 260 that the front-side thermistor 145a and the back-side thermistor 145b are formed, the user can use the resultant member without any discomfort.

When detecting defecation, the control circuit 108a of the automatic urine disposal apparatus 100 actuates the alarm lamp 504 for notification of the defecation. Therefore, when defecation has occurred, it is possible to notify the caregiver of the defecation, for example.

The automatic urine disposal apparatus 100, which also has the urine detection section 102b for detecting discharge of urine, can detect not only defecation but also urination.

Since the urine detection section 102b includes the paired urine detection electrodes 218a and 218b placed on the insulating synthetic resin film 260 with spacing therebetween, the function of detecting urine can be achieved at low cost. Also, since the paired urine detection electrodes 218a and 218b are placed on the thin, flexible, insulating synthetic resin film 260, the user can use the resultant member without any discomfort. Moreover, since the conductivity of the paired urine detection electrodes 218a and 218b with spacing therebetween increases with the presence of urine, detection of urine can be ensured by detecting urine based on a change in the voltage between the urine detection electrodes 218a and 218b.

Furthermore, since the automatic urine disposal apparatus 100 is provided with the vacuum suction device 100a that can suck urine, urine is sucked when urination is detected, permitting continuous use of the apparatus.

==Other Embodiments==

While the automatic urine disposal apparatus as the defecation detection apparatus of the present invention has been described with reference to the preferred embodiment, the embodiment has been presented for easy understanding of the present invention and is not intended to limit the invention. It is to be understood that changes and modifications can be made to the present invention without departing from the purport of the invention, and that the present invention should include any equivalents thereof.

While the automatic urine disposal apparatus 100 was described as an example of the defecation detection apparatus in the above embodiment, the defecation detection apparatus may have a form including neither the urine detection section nor the pump unit, for example. That is, it is only necessary to have a configuration including an electrode section that has the front-side thermistor 145a and the back-side thermistor 145b and has no urine detection section, a feces receiving member equivalent to the urine absorption member having no electrode section, and a control circuit connected to the electrode section for detecting whether defecation is present or not based on the signals output from the front-side thermistor 145a and the back-side thermistor 145b.

In the above embodiment, an example using two temperature sensors, i.e., the front-side thermistor and the back-side thermistor was described. The present invention is not limited to this. For example, a plurality of thermistors may be provided in the electrode section along the length direction, to detect signals output from the thermistors. When a temperature change where the temperature sharply rises and then slowly falls has been detected, a thermistor that has detected this temperature change may be specified as a defecation position thermistor placed at the defecation position, and a thermistor that is closest to the defecation position thermistor and has not detected the temperature change of sharp rise and slow fall may be specified as a non-defecation position thermistor placed at a non-defecation position. Whether defecation is present or not may be detected by subtracting the signal output from the non-defecation position thermistor from the signal output from the defecation position thermistor. In this case, with the non-defecation position thermistor placed closer to the defecation position, noise can be removed more correctly, permitting more correct detection of whether defecation is present or not.

Description of Reference Numerals

100 Automatic urine disposal apparatus (defecation detection apparatus)
100a Vacuum suction device (urine suction device)
101 Controller
102 Urine absorption member
102a Container section (feces receiving member)
102b urine detection section
102c Exposure portion
104 Joint member
106 Urine guide tube
106a Urine tank
108 Pump unit
108a Control circuit (control section)
108b Suction pump
112 Container
114 Urine drainage port
116 Electrical wiring
116a Power supply
118 Electrode section
120 Clip
124 Substantially non-air-permeable sheet
126 Diffusion sheet
128 Cushion sheet
130 Spacer
132 Filter
134 Skin-contact sheet
136 Leakage barrier
136a Sheet
136b Elastic member
136c Outer edge portion
136d Inner edge portion
138 End sheet
140 End sheet
143a Power supply electrode
143b Power supply electrode
143c Power supply electrode
145 Thermistor
145a Front-side thermistor (second temperature sensor)
145b Back-side thermistor (first temperature sensor)
150 Detection section
152 Peripheral flange
169a Uncoated portion
170 Insulating sheath
171 Opening
218a Urine detection electrode (paired electrode)
218b Urine detection electrode (paired electrode)
250 Break detection circuit
260 Film
265 Connecting portion
266 Top end portion
267a Side portion
267b Side portion
268 Bottom end portion
300 Underpants
301 Front waist region

301 Back waist region
303 Crotch region
504 Alarm lamp (Notification section)

**Claims**

1. A defecation detection apparatus comprising:

   a feces receiving member (102a) suitable to be placed to face a body of a wearer to receive discharged feces;
   a first temperature sensor (145b) placed at a defecation position of the feces receiving member where discharged feces are received;
   **characterized in that** it further comprises
   a second temperature sensor (145a) placed at a non-defecation position of the feces receiving member where discharged feces are not received; and
   a control section (108a) that is adapted to detect whether the discharged feces is present or not based on a signal output from the first temperature sensor and a signal output from the second temperature sensor.

2. A defecation detection apparatus according to claim 1, wherein
   the control section is adapted to detect whether the discharged feces is present or not based on a signal obtained by subtracting the signal output from the second temperature sensor from the signal output from the first temperature sensor.

3. A defecation detection apparatus according to claim 1 or 2, wherein
   the second temperature sensor is placed at a position facing the groin or a position between the position facing the groin and the defecation position when the feces receiving member faces the body.

4. A defecation detection apparatus according to any of claims 1 to 3, wherein
   the first temperature sensor and the second temperature sensor are formed on a single insulating synthetic resin film.

5. A defecation detection apparatus according to any of claims 1 to 4, further comprising:

   a notification section (504) that make a notification that feces have been discharged,
   wherein the control section is adapted to actuate the notification section when detecting that feces have been discharged.

6. A defecation detection apparatus according to any of claims 1 to 5, further comprising:

   a urine detection section (102b) that is adapted to detect urine discharged into the feces receiving member.

7. A defecation detection apparatus according to claim 6, wherein
   the urine detection section includes paired electrodes (218a, 218b) formed on the insulating synthetic resin film with spacing therebetween, and
   urine is detected based on a change in voltage between the paired electrodes caused by the discharged urine.

8. A defecation detection apparatus according to any of claims 1 to 7, further comprising:

   a urine suction device (100a) detachably attached to the feces receiving member, the urine suction device allowing sucking of urine discharged in the feces receiving member,
   wherein the control section is adapted to actuate the urine suction device to suck urine in the feces receiving member when the urine detection section has detected urine.

**Patentansprüche**

1. Stuhlentleerungsdetektor, der Folgendes umfasst:

   ein Stuhlaufnahmeelement (1 02a), das gegenüber einem Körper eines Trägers platziert werden kann, um den

13

ausgeschiedenen Stuhl aufzunehmen,
einen ersten Temperatursensor (145b), der an einer Entleerungsposition des Stuhlaufnahmeelements platziert wird, wenn der ausgeschiedene Stuhl aufgenommen wird, **gekennzeichnet dadurch, dass** es ferner Folgendes umfasst:

einen zweiten Temperatursensor (145a), der in eine Nicht-Stuhlentleerungsposition des Stuhlaufnahmeelements platziert wird, wo ausgeschiedener Stuhl nicht aufgenommen wird, und
einen Kontrollabschnitt (108a), der so adaptiert ist, dass er erkennt, ob der ausgeschiedene Stuhl vorhanden ist, oder nicht auf einer Signalausgabe vom ersten Temperatursensor und einer Signalausgabe vom zweiten Temperatursensor basiert.

2. Stuhlentleerungsdetektor nach Anspruch 1, wobei
der Kontrollabschnitt, der so adaptiert ist, wenn der ausgeschiedene Stuhl vorhanden ist, oder nicht auf einer Signalausgabe basiert, der durch Subtrahieren der Signalausgabe vom zweiten Temperatursensor von der Signalausgabe vom ersten Temperatursensor erhalten wird.

3. Stuhlentleerungsdetektor nach Anspruch 1 oder 2, wobei
der zweite Temperatursensor an einer Position platziert wird, die der Leistenbeuge zugewandt ist, oder an einer Position, die zwischen der Position, die der Leistenbeuge und der Stuhlposition zugewandt ist, wenn das Stuhlaufnahmeelement dem Körper zugewandt ist.

4. Stuhlentleerungdetektor nach einem der Ansprüche 1 bis 3, worin
der erste Temperatursensor und der zweite Temperatursensor auf einem einzelnen isolierenden synthetischen Harzfilm geformt sind.

5. Stuhlentleerungsdetektor nach einem der Ansprüche 1 bis 4, der ferner Folgendes umfasst:

einen Anzeigeabschnitt (504), der anzeigt, dass Stuhl ausgeschieden wurde,
wobei der Kontrollabschnitt so adaptiert ist, dass er den Anzeigeabschnitt auslöst, wenn erkannt wird, dass Stuhl ausgeschieden wurde.

6. Stuhlentleerungsdetektor nach einem der Ansprüche 1 bis 5, der ferner Folgendes umfasst:

einen Urinentleerungsabschnitt (1 02b), der so adaptiert ist, dass er Urin feststellt, der in das Stuhlaufnahmeelement abgegeben wurde.

7. Stuhlentleerungsdetektor nach Anspruch 6, wobei
der Urinentleerungsabschnitt, der paarweise Elektroden (218a, 218b) umfasst, am isolierenden synthetischen Harzfilm mit Abständen dazwischen geformt wird, und
Urin festgestellt wird, der auf einer Änderung der Spannung zwischen den paarweisen Elektroden basiert, die vom ausgeschiedenen Urin verursacht werden.

8. Stuhlentleerungsdetektor nach einem der Ansprüche 1 bis 7, der ferner Folgendes umfasst:

ein Urinansauggerät (100a), das abnehmbar am Stuhlaufnahmelement angebracht ist, wobei das Urinansauggerät das Ansaugen von Urin ermöglicht, der in das Urinaufnahmeelement ausgeschieden wurde,
wobei der Kontrollabschnitt so adaptiert wurde, dass er das Urinansauggerät auslöst, um Urin in dem Stuhlaufnahmeelement ansaugt, wenn der Urindetektor Urin festgestellt hat.

**Revendications**

1. Appareil de détection de défécation comportant :

un élément de réception de matières fécales (102a) approprié pour être placé de manière à être orienté vers le corps d'un utilisateur pour recevoir des matières fécales déchargées ;
un premier capteur de température (145b) placé au niveau d'une position de défécation de l'élément de réception de matières fécales où des matières fécales déchargées sont reçues ;

**caractérisé en ce qu'**il comporte par ailleurs

un deuxième capteur de température (145a) placé au niveau d'une position de non-défécation de l'élément de réception de matières fécales où des matières fécales déchargées ne sont pas reçues ; et

une section de commande (108a) qui est adaptée pour détecter si les matières fécales déchargées sont présentes ou non en fonction d'un signal émis en provenance du premier capteur de température et d'un signal émis en provenance du deuxième capteur de température.

**2.** Appareil de détection de défécation selon la revendication 1, dans lequel

la section de commande est adaptée pour détecter si les matières fécales déchargées sont présentes ou non en fonction d'un signal obtenu en soustrayant le signal émis en provenance du deuxième capteur de température du signal émis en provenance du premier capteur de température.

**3.** Appareil de détection de défécation selon la revendication 1 ou la revendication 2, dans lequel

le deuxième capteur de température est placé au niveau d'une position orientée vers l'aine ou au niveau d'une position entre la position orientée vers l'aine et la position de défécation quand l'élément de réception de matières fécales est orienté vers le corps.

**4.** Appareil de détection de défécation selon l'une quelconque des revendications 1 à 3, dans lequel

le premier capteur de température et le deuxième capteur de température sont formés sur un simple film de résine synthétique d'isolation.

**5.** Appareil de détection de défécation selon l'une quelconque des revendications 1 à 4, comportant par ailleurs :

une section de notification (504) qui avertit que des matières fécales ont été déchargées,

dans lequel la section de commande est adaptée pour actionner la section de notification quand il est détecté que des matières fécales ont été déchargées.

**6.** Appareil de détection de défécation selon l'une quelconque des revendications 1 à 5, comportant par ailleurs :

une section de détection d'urine (102b) qui est adaptée pour détecter de l'urine déchargée dans l'élément de réception de matières fécales.

**7.** Appareil de détection de défécation selon la revendication 6, dans lequel

la section de détection d'urine comprend des électrodes groupées par paire (218a, 218b) formées sur le film de résine synthétique d'isolation avec un espace entre elles, et

de l'urine est détectée en fonction d'un changement de tension entre les électrodes groupées par paire causé par l'urine déchargée.

**8.** Appareil de détection de défécation selon l'une quelconque des revendications 1 à 7, comportant par ailleurs :

un dispositif d'aspiration d'urine (100a) attaché de manière détachable sur l'élément de réception de matières fécales, le dispositif d'aspiration d'urine permettant l'aspiration de l'urine déchargée dans l'élément de réception de matières fécales,

dans lequel la section de commande est adaptée pour actionner le dispositif d'aspiration d'urine à des fins d'aspiration de l'urine dans l'élément de réception de matières fécales quand la section de détection d'urine a détecté de l'urine.

FIG. 1

FIG. 2

FIG. 3

FIG. 4

FIG. 5

FIG. 6

FIG. 7

FIG. 8

FIG. 9

FIG. 10

OUTPUT OF BACK-SIDE
THERMISTOR

OUTPUT OF FRONT-SIDE
THERMISTOR

NOISE-REMOVED DATA

FIG. 11

EP 2 491 898 B1

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002301098 A
- JP 2008048912 B